(19) Europäisches Patentamt
European Patent Office
Office européen des brevets

(11) Publication number: **0 225 990**
**A1**

(12) # EUROPEAN PATENT APPLICATION

(21) Application number: **86114444.2**

(22) Date of filing: **17.10.86**

(51) Int. Cl.[4]: **C 07 C 45/58**
C 07 C 45/28, C 07 C 49/255
C 07 D 303/22

(30) Priority: **18.10.85 IT 2255585**

(43) Date of publication of application:
**24.06.87 Bulletin 87/26**

(84) Designated Contracting States:
**AT BE CH DE ES FR GB LI NL SE**

(71) Applicant: **Montedison S.p.A.**
**31, Foro Buonaparte**
**I-20121 Milan(IT)**

(72) Inventor: **Venturello, Carlo, Dr.**
**135, via XXIII Marzo**
**I-28100 Novara(IT)**

(72) Inventor: **D'Aloisio, Rino**
**25, via Romentino**
**I-28100 Novara(IT)**

(72) Inventor: **Ricci, Marco, Dr.**
**5, via Brescia**
**I-28100 Novara(IT)**

(74) Representative: **Weinhold, Peter, Dr. et al,**
**Patentanwälte Dipl.-Ing. G. Dannenberg Dr. P. Weinhold**
**Dr. D. Gudel Dipl.-Ing. S. Schubert Dr. P. Barz**
**Siegfriedstrasse 8**
**D-8000 München 40(DE)**

(54) **Process for the preparation of phenylpropanones.**

(57) Process for the preparation of phenylpropanones having formula:

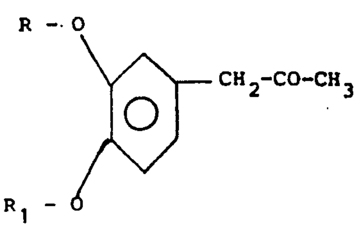

R and/or $R_1$, each other equal or different, being $C_1$-$C_4$ alkyl groups, optionally forming part of an alicyclic or heterocyclic ring, wherein the corresponding allylbenzenes are catalytically epoxidated by means of $H_2O_2$, in a biphase system comprising an aqueous phase containing $H_2O_2$, an organic phase containing said allylbenzenes, a solvent immiscible in said aqueous phase and a catalyst having formula $Q_3XW_4O_{24}$ (Q being a quaternary cation, containing hydroxarbylic groups having on the whole from 20 to 70 C atoms, and X being P or As) and wherein the thus obtained epoxides are isomerized by heating at 90°-150°C, in the presence of catalytic amounts of LiI.

EP 0 225 990 A1

The invention concerns a process for the preparation of phenylpropanones having formula:

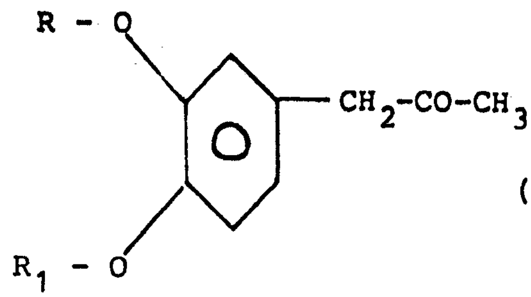

(I)

R and/or $R_1$, each other equal or different, are $C_1$-$C_4$ alkyl groups, optionally forming part of an alicyclic or heterocyclic ring, by catalytic epoxidation, by means of $H_2O_2$, of allylbenzenes having the formula:

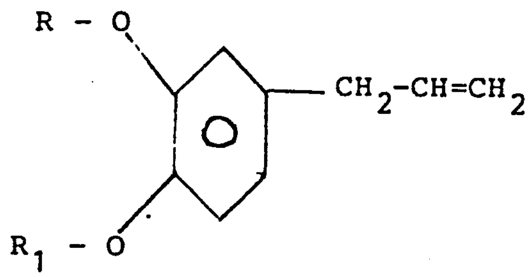

(II)

and subsequent catalytic isomerization of the thus obtained epo= xides to the corresponding phenylpropanones. Said phenylpropa= nones and particularly piperonylmethylketone (where R and $R_1$ are coincident in the $CH_2$ group of a heterocyclic ring) and veratryl= methylketone (where R = $R_1$ = $CH_3$), are useful intermediates for the synthesis of one of the most important antihypertensive agen= ts: alpha-methyl-beta- (3,4-dihydroxyphenyl)alanine (METHYLDOPA).

## BACKGROUND OF THE INVENTION

It was known to prepare phenylpropanones by oxidation (using peracids, such as performic or peracetic acid) of phenyl= propenes having formula:

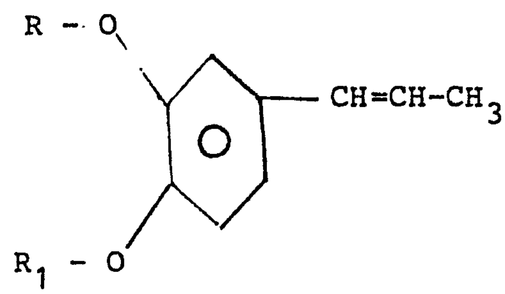

(V)

where R and $R_1$ have the same meaning as above to give an interme= diate glycol (via epoxide), which is successively converted into a ketone by a heat-treatment, in the presence of mineral acids. Such a process, however, gives rise to many a drawback; said pro= cess, infact:

- requires the use of an internal olefin (V), that is not retrie= vable on the market; therefore it has to be prepared by isome= rization of the corresponding end-olefin (II);
- requires the preparation of performic acid just at the moment when it has to be used, owing to its limited stability with ti= me ( a very thorough care is needed for safety purposes); al= ternatively use of expensive organic solutions of peracetic acid is needed;
- is bound up with problems that are connected with the recovery

or with the getting rid of considerable amounts of organic acids coming from the reduction of said peracids.

The Applicant has now found that it is possible to pre= pare said phenylpropanones by a simpler and cheaper catalytic pro= cess, free from danger and free from the other drawbacks of the known art, which process allows, in particular, to avoid the use of dangerous and/or expensive oxidizing agents, such as per formic or peracetic acid.

## DISCLOSURE OF THE INVENTION

In its widest form the invention concern a process com= prising the catalytic epoxidation, by means of $H_2O_2$, of allyl= benzenes (II) (without any preliminar isomerization to phenyl- -propenes) and subsequent catalytic isomerization of the obtai= ned epoxides to the corresponding phenylpropanones having for= mula (I).

More in detail our process is characterized in that:

a) the corresponding allylbenzene having formula (II) is epo= xidated by reaction with $H_2O_2$ under stirring, at 40° - 90°C, in a biphasic system comprising an aqueous phase, containing $H_2O_2$, and an organic phase consisting of at least one solvent immi= scible with the aqueous phase, of the allylbenzene having for= mula (II) and of a catalyst having general formula $Q_3 X W_4 O_{24}$ (IV),

wherein Q is a quaternary cation $(R_2R_3R_4R_5\ M)^+$, M being selected from the group comprising N, P and $R_2$, $R_3$, $R_4$ and $R_5$ (being the same or different) being selected from the group comprising hy= drogen and the hydrocarbylic groups (wherein said cation has from 1 to 4 hydrocarbylic groups containing, on the whole, from 20 to 70 carbon atoms) and wherein X is P or As, by using $H_2O_2$: allyl-benzene molar ratios between 1:1 and 1:2;

b) the organic phase coming from (a) (after removal of solvent and catalyst) or the epoxide isolated from said organic pha= se, having formula:

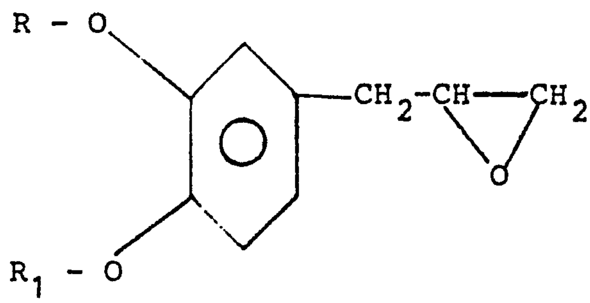

(III)

(R and $R_1$ having the same meaning as above) are isomerized by heating, under stirring, in the presence of catalytic amounts of lithium iodide, at temperatures from 90° to 150°C.

The main steps of the process according to the inven= tion may be summarized as follows:

1) 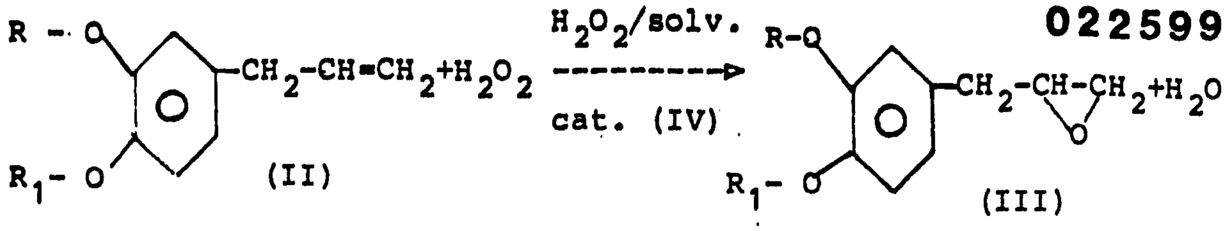


(II) (III)

2) 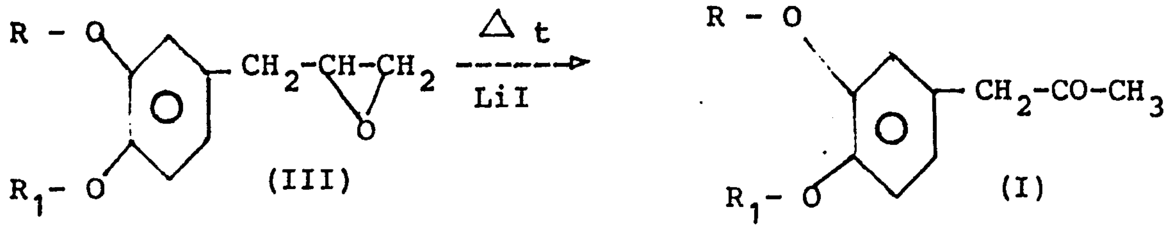


(III) (I)

Our phenylpropanones, in particular piperonylmethylketone and veratrylmethylketone, are obtained at a high purity level and with yields of 50-75% with respect to reacted allylbenzene (II).

Epoxydation catalyst IV consists of a peroxydic complex containing tungsten, phosphorus (or arsenic) and a sufficiently lipophilic quaternary cation, obtained according to known proces= ses. According to a preferred embodiment, X is phosphorus and in the quaternary cation M is nitrogen and $R_2$, $R_3$, $R_4$ and $R_5$ are hy= drocarbylic groups containing on the whole from 25 to 40 C atoms, such as in the case of methyltrioctylammonium, dimethyldioctade= cylammonium, dimethyldihexadecylammonium and mixtures thereof. Most preferred, are the catalysts having following formulas:

$(C_{25}H_{54}N)_3PW_4O_{24}$ and $(C_{37}H_{78}N)_3PW_4O_{24}$

(VI) (VII)

Such catalysts can be prepared, for instance, by reacting

tungstic acid (or an alkali metal tungstate), phosphoric acid (or an alkali metal phosphate) and hydrogen peroxide (in an aqueous and acid phase) with a quaternary salt, selected from the group comprising methyltrioctylammonium chloride (commercially traded as ALIQUAT 336) and dimethyl [dioctadecyl (75%) + dihexadecyl (25%)] ammonium chloride (commercially traded as ARQUAD 2HT), contained in an organic phase that is immiscible in the aqueous one. The reaction between the inorganic reactants may be carried out at 20 - 80°C; then the quaternary salt, dissolved in a solvent (for instance, 1,2-dichloroethane), is added, preferably at room temperature and the stirring of the biphasic mixture is carried on for 15-30 minutes. The aqueous and acid phase has preferably a pH below 2 and same pH can be adjusted by means of a mineral acid (for instance $H_2SO_4$ or HCl). The molar ratios among the reactants should generally be as follows: 4 moles of W and up to 2 moles of quaternary salt per each atom gram of P and from 2.5 to 6 moles of $H_2O_2$ per mole of W. After evaporation of the organic phase, compound (VI) or compound (VII) are obtained in an oily or solid form, respectively.

Epoxydation reaction (a) is then carried out according to the double phase technique; the organic phase contains allylbenzene (II), catalyst (IV) and a solvent immiscible with the aqueous phase.

Optionally substituted chlorinated hydrocarbons (for instance trichloromethane, tetrachloroethylene, dichloroethanes, trichloroethanes and so on) or aromatic hydrocarbons (for instance benzene, toluene or xylenes) can be used as immiscible solvents.

Our advise is to work under strong stirring at 40° - 90°C, preferably between 60° and 75°C, at atmospheric pressure; the reaction time (depending on the used catalyst and its amounts, on the temperature and on the nature and concentration of the allylbenzene) generally ranges between 2 and 3 hours; the catalyst:$H_2O_2$ molar ratio can range between 1:150 and 1:230. Finally a $H_2O_2$: substratum (II) molar ratio between 1:1 and 1:2 (preferably between 1:1.5 and 1:1.6) should be generally used. The amount of substratum (II) in the organic phase should generally range from 30 to 80% and preferably from 40 to 60% by weight. Use can be made of a concentration of $H_2O_2$, in the aqueous phase, between 1 and 70% and preferably between 10 and 30% by weight; a 98-99% $H_2O_2$ conversion is thus obtained.

The isomerization step (b) can be carried out on the organic phase as such, coming out from the epoxidation reaction (a), after solvent and catalyst (IV) have been removed, or the epoxide (III), can be isolated from said organic phase and then isomerized. Our advise is to work, under stirring, at 90 - 150° C, pre=

ferably at 130°C, and at atmospheric pressure. The reaction time, depending on the used catalyst, on its amount and on the tempera= ture, generally should range from 1 to 3 hours.

The amount of lithium iodide catalyst can range from 0.3 to 3%, preferably from 0.5 to 2% by weight, with respect to the organic phase to be isomerized, or in the case of isomeriza= tion of isolated epoxide (III), according to a LiI:epoxide molar ratio from 0.5:50 to 5:50, preferably from 1:50 to 3:50. When the reaction is over, the phenylpropanone can be isolated from the reaction medium by distillation, by elution or according to other usual techniques.

A few examples will follow by way of illustration, without limiting however the scope of the invention.

The hydrogen peroxide and phosphoric acid concentrations are expressed in the examples as grams per 100 $cm^3$ of solution.

EXAMPLE 1

a) Preparation of catalyst (VI), namely of $(C_{25}H_{54}N)_3\ PW_4O_{24}$
The preparation was exactely identical to the one described by example 3 of European Patent Application 86/109120.

b) Piperonylmethylketone preparation.
7 $cm^3$ of $H_2O$, 6.83 $cm^3$ of $H_2O_2$ at 40% (80 mmoles), 0.8 g (0.35 mmoles) of catalyst (VI) dissolved in 20 $cm^3$ of 1,2--dichloroethane, 20-30 mg of p-ter-butylphenol and 19.9 g of

98% safrole (120 mmoles) were loaded into a 3 neck flask having a 100 $cm^3$ capacity and equipped with blade stirrer, thermometer and reflux cooler. The biphasic mixture was brought, under fairly strong stirring, up to 60°C and kept at this temperature for 2 hours. A conversion of $H_2O_2$ higher than 98% was obtained (determined by iodometric titration of the aqueous phase). The organic phase (lower one) was separated, diluted with ethyl ether (30-40 $cm^3$), then kept under stirring for 5 minutes in contact with an aqueous solution (10 $cm^3$) containing 1 g of $Na_2SO_3$ and 1 g of $Na_2CO_3$ and then quickly eluted with ethyl ether on a small silica column. 400 mg of LiI were added to the light yellow oil obtained after the solvent evaporation and the resulting mixture was kept under stirring at 130°C (temperature of the thermostatic bath) for 1 hour; after cooling, the mixture was fractionated on a silica column (eluent: 50/50 mixture of ethyl ether and n--hexane). 8.50 g (47.75 mmoles) of piperonylmethylketone were thus obtained and 8.70 g (53.70 mmoles) of non-reacted safrole were recovered; selectivity to ketone, with respect to the (converted) safrole, was 72%.

EXAMPLE 2

Veratrylmethylketone preparation.

14 $cm^3$ of $H_2O$, 6.83 $cm^3$ of $H_2O_2$ at 40% (80 mmoles), 1.14 g (0.5 mmoles) of catalyst (VI) dissolved in 20 $cm^3$ of 1,2-dichloroethane and 20-30 mg of p-ter-butylphenol and 21.8 g of 98% methyleugenol (120 mmoles) were loaded into the flask of example 1. The biphasic mixture was brought up to 60°C, under fairly strong stirring, and kept at this temperature for 2.5 hours; a 98% conversion of $H_2O_2$ was obtained (determined by iodometric titration of the aqueous phase). The organic phase (lower one) was separated, diluted with ethylether (30-40 $cm^3$), then kept under stirring for 5 minutes in contact with an aqueous solution (10 $cm^3$) containing 1 g of $Na_2SO_3$ and 1 g of $Na_2CO_3$ and then eluted as in the first example. 400 mg of LiI were added to the light yellow oil obtained by evaporation of the solvent (21.4 g) and the resulting mixture was kept under stirring at 130°C (temperature of thermostatic bath) for 1 hour; the mixture was then cooled and fractionated as in the first example. 6.52 g (33.60 mmoles) of veratrylmethylketone were obtained and 10.18 g (57.2 mmoles) of non-reacted methyl eugenol were recovered. Selectivity to ketone (with respect to the converted methyl eugenol) was 53.5%.

EXAMPLE 3

a) Preparation of catalyst (VII), namely of $(C_{37}H_{78}N)_3PW_4O_{24}$

The preparation was exactely identical to the one of example 1 of European Patent Application 86/109120.

b) Veratrylmethylketone preparation.

Working as in example 2, using 2 g (about 0.7 mmoles) of catalyst (VII), instead of catalyst (VI), and keeping the biphasic mixture at 70°C for 1 hour (instead of 60°C for 2.5 hours), 6.01 g (30.97 mmoles) of veratrylmethylketone were obtained and 10.53 g (59.2 mmoles) of non-reacted methyl eugenol were recovered. Selectivity was slightly lower (50.94%).

WHAT IS CLAIMED IS:

1. A process for the preparation of a phenylpropanone having formula (I), characterized in that:

a) the corresponding allyl-benzene having formula (II) is epoxidated, by reaction with $H_2O_2$, under strong stirring, at 40° - 90° C, in a biphasic system comprising an aqueous phase containing $H_2O_2$ and an organic phase consisting of at least one solvent immiscible in the aqueous phase, of said allyl-benzene and of a catalyst having general formula $Q_3XW_4O_{24}$ (IV), wherein Q is a quaternary cation $(R_2R_3R_4R_5\ M)^+$, M being selected from the group comprising N and P and $R_2$, $R_3$, $R_4$ and $R_5$ (same or different) being selected from the group comprising hydrogen and hydrocarbylic groups, in order to have from 1 to 4 hydrocarbylic groups containing, on the whole, from 20 to 70 C atoms, and wherein X is P or As, by using $H_2O_2$:allylbenzene molar ratios between 1:1 and 1:2;

b) the organic phase coming from (a), after having removed the solvent and catalyst, or the epoxide having formula:

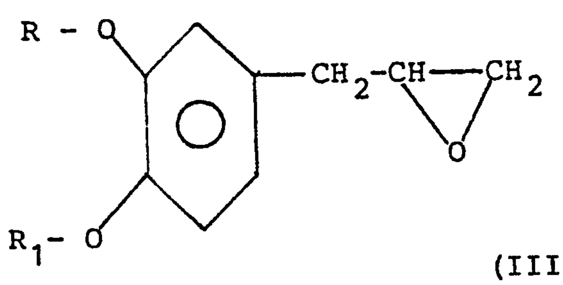

(III)

wherein $R_1$ and $R_2$ have the same meanings as specified hereinbefore, after having been isolated from said organic phase, is isomerized by heating, under stirring, in the presence of catalytic amounts of lithium iodide, at temperatures ranging between 90° and 150°C.

2. A process according to claim 1, wherein R and/or $R_1$ represent a $CH_3$ group.

3. A process according to one or both of the claims 1-2, characterized in that in catalyst (IV) X represents phosphorus and in the $(R_2R_3R_4R_5M)^+$ cation M represents nitrogen and $R_2$, $R_3$, $R_4$ and $R_5$ represent hydrocarbylic groups containing, on the whole, from 25 to 40 C atoms.

4. A process according to claim 3, wherein said cation is selected from the group comprising methyltrioctylammonium, dimethyldioctadecylammonium, dimethyldihexadecylammonium or mixtures thereof.

5. A process according to one or more of the claims 1-4, wherein catalyst (IV) is selected from the compounds having formula:

$(C_{25}H_{54}N)_3\ P\ WO_4O_{24}$ and $(C_{37}H_{78}N)_3P\ W_4O_{24}$

(VI) (VII)

6. A process according to one or more of the claims 1-5, wherein the solvent immiscible with the aqueous phase is selected from the group consisting of optionally substituted chlorinated and aromatic hydrocarbons.

7. A process according to one or more of the claims 1-6, wherein the epoxidation temperature ranges between 60° and 75°C, the $H_2O_2$:allylbenzene molar ratio ranges from 1:1.5 to 1:1.6 and the catalyst:$H_2O_2$ molar ratio ranges from 1:150 to 1:230.

8. A process according to one or more of the claims 1-7, wherein the amount of allylbenzene in the organic phase ranges from 40% to 60% by weight.

9. A process according to one or more of the claims 1-8, wherein the amount of $H_2O_2$ in the aqueous phase ranges from 10% to 30% by weight.

10. A process according to one or more of the claims 1-9, wherein lithium iodide is used in an amount from 0.3 to 3% by weight with respect to the organic phase to be isomerized or according to a LiI:epoxide (III) molar ratio from 0.50:50 to 5:50.

11. A process according to one or more of the claims 1-10, wherein the isomerization temperature is 130°C.

European Patent Office

# EUROPEAN SEARCH REPORT

Application number

EP 86 11 4441

0225990

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | EP-A-0 109 273 (MONTEDISON S.p.A.)<br>* Whole document * | 1-9 | C 07 C 45/58<br>C 07 C 45/28<br>C 07 C 49/255<br>C 07 D 303/22 |
| X | GB-A-2 055 821 (INSTITUTO GUIDO DONEGANI)<br>* Whole document * | 1-9 | |
| Y | FR-A-2 300 765 (PRODUITS CHIMIQUES UGINE KUHLMANN)<br>* Whole document * | 1-9 | |
| Y | FR-A-2 204 369 (DYNAPOL)<br>* Example 1 * | 1-9 | |
| X | RESEARCH DISCLOSURE, November 1973, pages 5,6, abstract no. 11504: "Isomerization of epoxy compounds (C07c)"<br>* Page 5, column 2 * | 1,10,11 | **TECHNICAL FIELDS SEARCHED (Int. Cl.4)**<br>C 07 C 45/00<br>C 07 C 49/00<br>C 07 D 303/00<br>C 07 D 301/00 |
| Y | GB-A- 905 821 (CHEMISCHE WERKE HÜLS AG)<br>* Page 1; claims * | 1,10,11 | |
| Y | DE-A-1 793 570 (MERCK & CO.)<br>* Page 2 * | 1,10,11 | |

--- -/-

The present search report has been drawn up for all claims

| Place of search | Date of completion of the search | Examiner |
|---|---|---|
| THE HAGUE | 09-03-1987 | BONNEVALLE E.I.H. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

European Patent Office

# EUROPEAN SEARCH REPORT

Application number
**0225990**

EP 86 11 4444

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DE-A- 174 496 (P. HÖRING)<br>* Examples 1,3 *<br>----- | 1,10, 11 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 09-03-1987 | BONNEVALLE E.I.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82